# EUROPEAN PATENT APPLICATION

(11) **EP 4 803 071 A1**
(43) Date of publication of application: **09.09.2026**
(21) Application number: 24891742.9
(22) Date of filing: 12.11.2024
(51) Int. Cl.: A61K 9/00, A61K 47/38, A61K 47/32, A61K 47/36, A61M 37/00, A61K 9/70

(54) **MICRONEEDLE PATCH AND ADHESIVE COMPOSITION**

(30) Priority: 16.11.2023 KR 20230158990
(71) Applicant: Raphas Co., Ltd., Seoul 07793 (KR)
(72) Inventor: KIM, Seong-Jun, Anyang-si Gyeonggi-do 14040 (KR); LA, Sookie, Seoul 08023 (KR); YOO, Na-Young, Incheon 22627 (KR); JEONG, Do-Hyeon, Goyang-si Gyeonggi-do 10543 (KR)
(74) Representative: Prüfer & Partner mbB Patentanwälte · Rechtsanwälte
(86) International application number: PCT/KR2024/017809
(87) International publication number: WO 2025/105796

(57) **Abstract**

The present invention relates to a microneedle patch and an adhesive composition, and more particularly, to a microneedle patch and an adhesive composition in which the microneedles may be directly attached to an adhesive sheet without a support film for supporting hydrophilic microneedles when providing the microneedle patch.

## Description

### [Technical Field]

The present invention relates to a microneedle patch and an adhesive composition, and more particularly, to a microneedle patch and an adhesive composition in which the microneedles may be directly attached to an adhesive sheet without a support film for supporting hydrophilic microneedles when providing the microneedle patch.

### [Background Art]

Although numerous drugs and physiologically active substances for treating diseases have been developed, problems of passing through biological barriers (biological barriers such as skin, oral mucosa, and brain-blood vessel barriers) and problems of efficiency of drug delivery in delivering drugs and physiologically active substances into the body still remain to be improved.

Drugs and physiologically active substances are generally administered orally in tablet form or capsule form, but cannot be effectively delivered by such an administration method alone due to reasons such as numerous drugs being digested or absorbed in the gastrointestinal tract or being lost by a liver mechanism. Furthermore, some drugs cannot be effectively diffused through the mucous membrane of the intestine. In addition, patient compliance is also a problem (for example, in the case of patients who need to take drugs at specific intervals, or critically ill patients who cannot take drugs).

Another conventional technique in delivering drugs and physiologically active substances is the use of a conventional needle. While this method is effective compared to oral administration, it has problems of causing pain at an injection site and causing local skin damage, bleeding, and disease infection at the injection site.

In order to solve the above-described problems, various microneedle patches comprising microneedles have been developed. Microneedle patches developed to date have been mainly used for in-vivo drug delivery, blood collection, and detection of analytes in the body. Metals and various polymer materials have been used as the material of the microneedles. Recently, biodegradable polymer materials have been gaining attention as the material of microneedles.

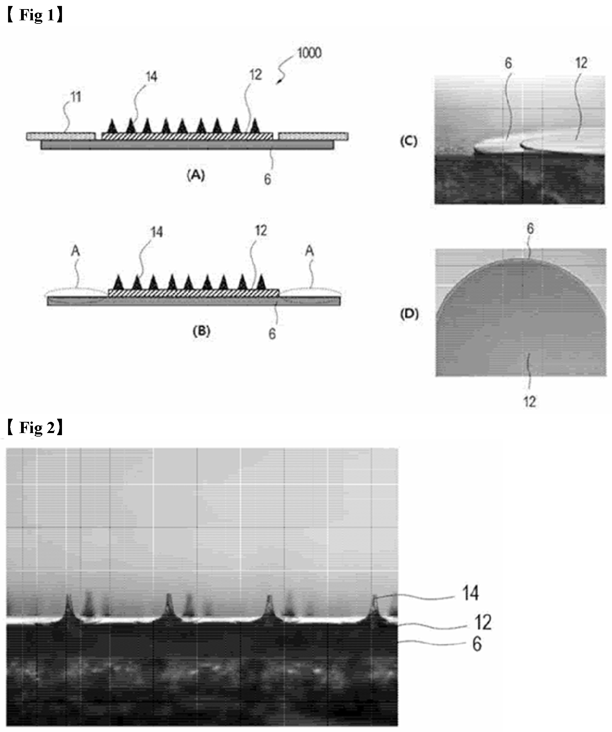

FIG. 1 corresponds to a drawing and a photograph showing a microneedle patch 1000 comprising microneedles 14 according to the prior art. FIG. 1A is a side cross-sectional view of the microneedle patch 1000 according to the prior art, FIG. 1B shows a state in which a protective release film 11 has been removed from the microneedle patch 1000, FIG. 1C is a photograph showing an adhesive sheet 6 and a support film 12 provided on an upper surface of the adhesive sheet 6 from the side, and FIG. 1D is a plan photograph of the adhesive sheet 6 and the support film 12 viewed from above.

Referring to FIG. 1, the microneedle patch 1000 comprises an adhesive sheet 6, a support film 12 provided on an upper surface of the adhesive sheet 6, and microneedles 14 provided on an upper surface of the support film 12. Furthermore, the microneedle patch 1000 may further comprise a protective release film 11 disposed on the upper surface of the adhesive sheet 6 so as to surround the support film 12.

The adhesive sheet 6 provides an adhesive force so that the microneedles 14 are in close contact with the skin of a user. The adhesive sheet 6 is covered by the aforementioned protective release film 11 before using the microneedle patch 1000, and when in use, the user removes the protective release film 11 (the state of FIG. 1B) to expose the adhesive sheet 6.

The support film 12 is provided on the upper surface of the adhesive sheet 6 and serves as a support on which the microneedles 14 are formed. The microneedles 14 may be made of a hydrophilic or water-soluble material. In addition, the adhesive sheet 6 may be made of a hydrophobic material to maintain adhesive force.

In this case, since the hydrophilic microneedles 14 are not attached to the hydrophobic adhesive sheet 6 with sufficient bonding force, a hydrophilic support film 12 is first formed or attached to the upper surface of the adhesive sheet 6 in the prior art, and the microneedles 14 are then formed on the upper surface of the support film 12. FIGS. 1C and 1D illustrate a state in which the support film 12 is provided on the upper surface of the adhesive sheet 6.

When the hydrophilic support film 12 is thus provided, the microneedles 14 may be formed on the aforementioned support film 12, as shown in FIG. 2.

In the case of the microneedle patch 1000 according to the prior art having the above-described structure, as described above, the protective release film 11 is removed to expose the adhesive sheet 6 during use. However, since the support film 12 for forming the microneedles 14 is provided on the upper surface of the adhesive sheet 6, the adhesive sheet 6 actually exposed corresponds to an edge area A of the upper surface of the adhesive sheet 6, as shown in FIG. 1B.

That is, when the microneedle patch 1000 according to the prior art is attached to the skin of the human body, only the edge area A of the upper surface of the adhesive sheet 6 is attached to the human body, and the adhesive force does not directly act on the region of the support film 12 on which the microneedles 14 are formed since the adhesive sheet 6 is not exposed. As a result, in the structure according to the prior art, there was a problem in that the adhesive force for attaching the microneedles 14 to the skin is insufficient, causing the microneedles 14 to not be in close contact with the skin, thereby significantly deteriorating drug delivery capability.

### [Disclosure of Invention]

### [Technical Problem]

The present invention aims to provide a microneedle patch capable of bringing microneedles into close contact with the skin when attaching the microneedle patch to the skin of a user, in order to solve the above-described problems.

In addition, the present invention aims to provide an adhesive composition to which hydrophilic microneedles may be directly connected.

### [Solution to Problem]

The above-described object of the present invention is achieved by a microneedle patch comprising an adhesive sheet and hydrophilic microneedles provided on the adhesive sheet, wherein the microneedles are directly connected to one surface of the adhesive sheet.

Here, the hydrophilic microneedles may be configured as a plurality of microneedles, and the adhesive sheet may be exposed between the plurality of microneedles.

Furthermore, the adhesive sheet may comprise a hydrophilic polymer.

Meanwhile, the adhesive sheet comprises an adhesive layer formed of an adhesive composition comprising the hydrophilic polymer and an adhesive and to which the microneedles are connected, and a film layer provided below the adhesive layer, and the adhesive composition may comprise 5 to 40 wt% of the hydrophilic polymer.

In addition, the hydrophilic polymer may be composed of one type or a mixture of two or more types selected from CMC (carboxymethyl cellulose), Carbomer, HPMC (hydroxypropyl methyl cellulose), PVP (polyvinyl pyrrolidone), PVA (polyvinyl alcohol), and Guar Gum.

Meanwhile, the adhesive may comprise 10 to 50 wt% of synthetic rubber, 30 to 70 wt% of hydrogenated hydrocarbon resin, 5 to 20 wt% of mineral oil, and 0.2 to 3 wt% of an antioxidant.

Furthermore, a contact angle of the adhesive sheet may be 70° to 100°.

In addition, a viscosity of the hydrophilic polymer may be 5 mPa·s to 5,000 mPa·s.

Meanwhile, the above-described object of the present invention may be achieved by an adhesive composition for forming an adhesive layer of an adhesive sheet included in a microneedle patch, wherein the composition comprises a hydrophilic polymer.

Furthermore, the hydrophilic polymer may be composed of one type or a mixture of two or more types selected from CMC (carboxymethyl cellulose), Carbomer, HPMC (hydroxypropyl methyl cellulose), PVP (polyvinyl pyrrolidone), PVA (polyvinyl alcohol), and Guar Gum.

In addition, the hydrophilic polymer may be comprised in the composition at 5 to 40 wt%.

Meanwhile, the composition comprises an adhesive and the hydrophilic polymer, and the adhesive may comprise 10 to 50 wt% of synthetic rubber, 30 to 70 wt% of hydrogenated hydrocarbon resin, 5 to 20 wt% of mineral oil, and 0.2 to 3 wt% of an antioxidant.

In this case, a viscosity of the hydrophilic polymer may be 5 mPa·s to 5,000 mPa·s.

### [Advantageous Effects of Invention]

According to the present invention having the above-described configuration, when attaching the microneedle patch to the skin of a user, the adhesive sheet between the microneedles is exposed, thereby allowing the microneedles to be effectively brought into close contact with the skin.

In addition, according to the present invention, by manufacturing an adhesive sheet using an adhesive composition comprising a hydrophilic polymer, microneedles may be directly connected to one surface of the adhesive sheet.

### [Brief Description of Drawings]

FIG. 1 is a drawing showing a microneedle patch according to the prior art,
FIG. 2 is a photograph of microneedles in FIG. 1,
FIG. 3 is a drawing showing a microneedle patch according to an embodiment of the present invention,
FIG. 4 is a photograph of microneedles in FIG. 3,
FIG. 5 is a drawing showing a method of manufacturing a microneedle patch according to an embodiment of the present invention by a blowing-stretching method,
FIG. 6 is a drawing schematically showing a method of manufacturing a microneedle patch having the configuration according to FIG. 3 by a mold method,
FIG. 7 is a side cross-sectional view showing the configuration of an adhesive layer,
FIG. 8 is a photograph of microneedles formed on an adhesive sheet comprising various hydrophilic polymers,
FIG. 9 is a photograph of a state in which microneedles are not accurately formed,
FIG. 10 is a photograph showing whether microneedles are formed according to changes in contact angle depending on the content of a hydrophilic polymer in the adhesive sheet according to the present invention and the adhesive sheet according to the prior art when a microneedle patch is manufactured by the blowing-stretching method,
FIG. 11 is a photograph of the degree of formation of microneedles depending on the viscosity of the hydrophilic polymer when a microneedle patch is manufactured by the blowing-stretching method.

### [Best Mode]

Hereinafter, a microneedle patch according to an embodiment of the present invention will be described in detail with reference to the drawings.

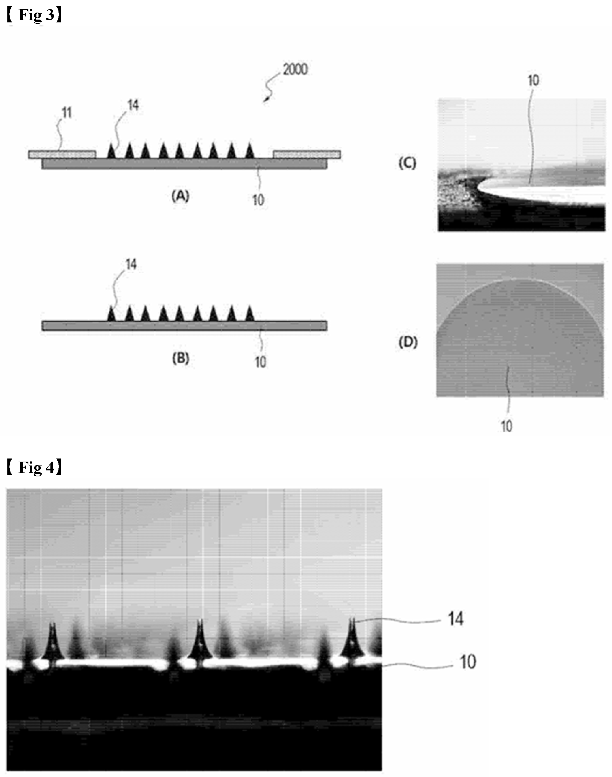

FIG. 3 is a drawing showing a microneedle patch 2000 according to an embodiment of the present invention, and FIG. 4 corresponds to an actual photograph of the microneedles 14 in FIG. 3. FIG. 3A is a side cross-sectional view of the microneedle patch 2000 according to the present invention, FIG. 3B is a side cross-sectional view showing a state in which the protective release film 11 has been removed from the microneedle patch 2000, FIG. 3C is a photograph showing the adhesive sheet 10 from the side, and FIG. 3D corresponds to a plan photograph of the adhesive sheet 10 viewed from above.

Referring to FIGS. 3 and 4, the microneedle patch 2000 may comprise an adhesive sheet 10 and microneedles 14 provided on the adhesive sheet 10.

Here, the microneedles 14 are inserted into the skin and dissolved, and may be made of a biocompatible and biodegradable material and a water-soluble or hydrophilic material.

For example, the microneedles 14 may be made of hyaluronic acid and salts thereof, polyvinyl pyrrolidone, polyvinyl alcohol, a cellulose polymer, dextran, gelatin, glycerin, polyethylene glycol, polysorbate, propylene glycol, povidone, carbomer, gum ghatti, guar gum, glucomannan, glucosamine, dammer resin, rennet casein, locust bean gum, microfibrillated cellulose, psyllium seed gum, xanthan gum, arabino galactan, gum arabic, alginic acid, gelatin, gellan gum, carrageenan, karaya gum, curdlan, chitosan, chitin, tara gum, tamarind gum, tragacanth gum, furcelleran, pectin or pullulan, hydroxypropyl methylcellulose, hydroxyalkyl cellulose, ethyl hydroxyethyl cellulose, alkyl cellulose, and carboxymethyl cellulose.

Meanwhile, in the present invention, the microneedles 14 may be directly connected to or formed on the upper surface or one surface of the adhesive sheet 10. That is, the microneedles 14 may be directly formed on or attached to the upper surface of the adhesive sheet 10. Referring to FIGS. 3C and 3D, it can be confirmed that there is no hydrophilic support film as in the prior art on the upper surface of the adhesive sheet 10. In addition, as shown in FIG. 4, it can be confirmed that the microneedles are normally attached and provided on the upper surface of the adhesive sheet 10 without a hydrophilic support film.

In this way, when the microneedles 14 are directly provided on the upper surface of the adhesive sheet 10 and the microneedles 14 are configured as a plurality of microneedles, the adhesive sheet 10 between the plurality of microneedles 14 may be exposed, as shown in FIGS. 3A and 3B.

That is, when a user removes the protective release film 11 as shown in FIG. 3B in order to use the microneedle patch 2000, not only the edge area of the adhesive sheet 10 but also the adhesive sheet 10 between the microneedles 14 may be exposed.

Therefore, when the upper surface of the adhesive sheet 10 provided with the microneedles 14 is attached to the skin of the human body, the adhesive sheet 10 is also exposed between the microneedles 14 together with the edge area of the adhesive sheet 10, thereby allowing the microneedles 14 to be more effectively brought into close contact with the skin of the human body. In this case, drugs and the like may be more effectively delivered into the human body through the microneedles 14, thereby greatly enhancing the drug delivery capability of the microneedle patch 2000.

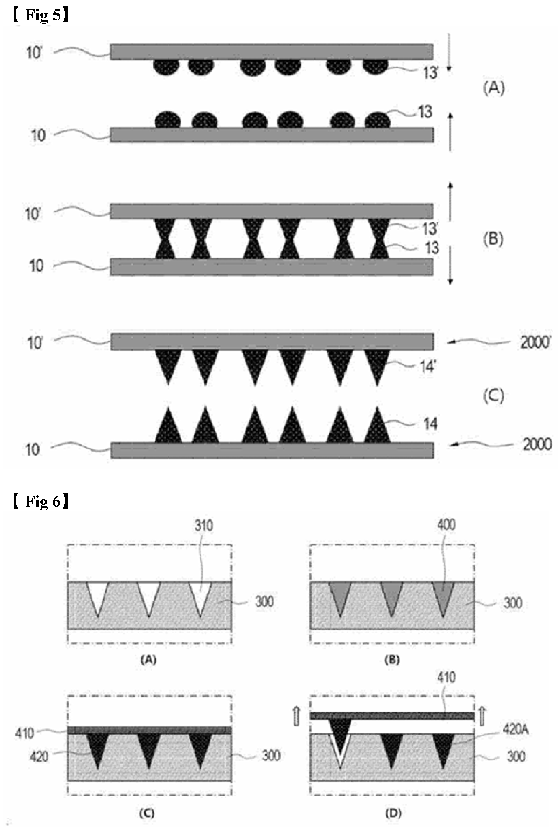

Meanwhile, FIG. 5 is a drawing showing a method of manufacturing the microneedle patches 2000, 2000' according to the present invention. The method corresponds to a blowing-stretching (DEN, Droplet Extension) method developed by the present applicant. For microneedles manufactured by the blowing-stretching method, reference may be made to Korean Registered Patent Nos. 1254240, 1285085, 1636069, 1816922, 2103194, and 2127123, and the entire contents of these patents may be incorporated herein by reference.

Referring to FIG. 5, the step of manufacturing the microneedle patches 2000, 2000' may first comprise a step of providing a pair of adhesive sheets 10, 10' and a step of spotting biodegradable viscous materials 13, 13' on at least one of the pair of adhesive sheets 10, 10'.

The adhesive sheets 10, 10' may, for example, be provided on a pair of substrates (not shown). In this case, the adhesive sheets 10, 10' may be provided by being applied and dried on the substrates, or may be provided in a sheet state.

In the present invention, as described above, the hydrophilic support film may be omitted on the mutually opposing surfaces of the adhesive sheets 10, 10'. As a result, the microneedles 14, 14' may be directly formed on the mutually opposing surfaces (upper surfaces) of the adhesive sheets 10, 10'.

Meanwhile, although FIG. 5A shows the viscous materials 13, 13' as being spotted on both of the pair of adhesive sheets 10, 10', the present invention is not limited thereto. For example, it is also possible to spot the viscous materials 13, 13' on only one of the pair of adhesive sheets 10, 10'.

Subsequently, the pair of adhesive sheets 10, 10' are relatively moved (FIG. 5A) so as to approach each other, the viscous materials 13, 13' are brought into contact with each other between the pair of adhesive sheets 10, 10', and the pair of adhesive sheets 10, 10' are separated to stretch the viscous materials 13, 13' (FIG. 5B).

When the pair of adhesive sheets 10, 10' are separated from each other in this way, if the bonding force between the viscous materials 13, 13' forming the microneedles and the pair of adhesive sheets 10, 10' is weakened, the viscous materials 13, 13' may not be stretched in the stretching step of FIG. 5B and may be separated from the pair of adhesive sheets 10, 10'. In the present invention, by including a hydrophilic polymer in the adhesive sheets 10, 10', the bonding force between the viscous materials 13, 13' forming the microneedles and the pair of adhesive sheets 10, 10' is maintained.

That is, the adhesive force between the viscous materials 13, 13' and the pair of adhesive sheets 10, 10' may be determined to a degree at which the viscous materials 13, 13' are able to form a tip portion, or more.

Subsequently, the viscous materials 13, 13' are solidified and the pair of adhesive sheets 10, 10' are separated to directly form the microneedles 14, 14' on the pair of adhesive sheets 10, 10'.

In this case, the viscous materials 13, 13' may be solidified by blowing or the like. When the pair of adhesive sheets 10, 10' are further separated after the viscous materials 13, 13' are sufficiently solidified, the viscous materials 13, 13' that were connected to each other are separated, thereby forming the microneedles 14, 14' having tip portions, and the microneedle patches 2000, 2000' are completed (FIG. 5C).

Meanwhile, as described above, since the microneedles 14 are water-soluble or hydrophilic, if the adhesive sheet 10 is hydrophobic as in the prior art, the bonding force between the microneedles 14 and the adhesive sheet 10 becomes very low. In this case, when manufacturing the microneedle patch 2000 by the blowing-stretching method according to FIG. 5 described above, when the adhesive sheets 10 are separated to both sides, the viscous materials 13, 13' between the adhesive sheets 10 may not be stretched to both sides, and the viscous materials 13, 13' may be separated from one of the adhesive sheets 10, so that the microneedles 14 may not be formed.

Such a problem may occur not only in the method of manufacturing a microneedle patch according to the above-described blowing-stretching method but also in the case of manufacturing by a mold method. FIG. 6 schematically illustrates a method of manufacturing a microneedle patch having the configuration according to FIG. 3 by the mold method.

Referring to FIG. 6, when manufacturing a microneedle patch by the mold method, a needle composition 400 is placed in cavities 310 of a mold 300, an adhesive sheet 410 is provided on the top thereof, and the needle composition 400 is dried through drying or appropriate heat treatment. Subsequently, when the needle composition 400 is dried, the adhesive sheet 410 is pulled to separate it from the mold 300.

In this way, when the adhesive sheet 410 is separated from the mold 300, if the bonding force between the adhesive sheet 410 and the microneedles 420 is weakened, some microneedles 420A among the microneedles 420 may not be separated from the mold 300, and only the adhesive sheet 410 may be separated.

In the present invention, an adhesive sheet and an adhesive composition are provided that maintain the bonding force between the adhesive sheet 10 and the hydrophilic microneedles 14 to a degree sufficient to manufacture the microneedle patch 2000, and furthermore, maintain adhesive force so that the adhesive sheet 10 may be firmly attached to the skin of the human body. This will be described in detail below.

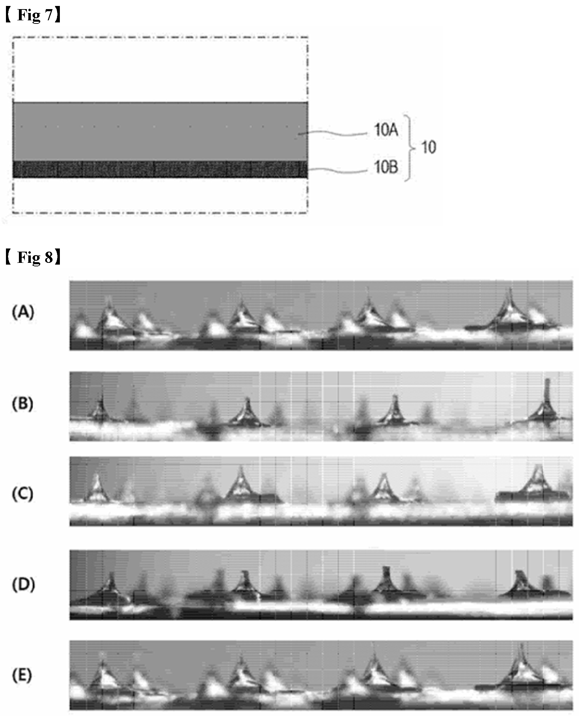

FIG. 7 is a side cross-sectional view showing the adhesive sheet 10 according to the present invention. In FIG. 7, the microneedles 14 are shown in an omitted state.

Referring to FIG. 7, the adhesive sheet 10 may comprise an adhesive layer 10A and a film layer 10B provided below the adhesive layer 10A. The aforementioned microneedles 14 are provided on the upper surface of the adhesive layer 10A.

The film layer 10B may, for example, comprise at least one or more of a PET (polyester) film and a PU (polyurethane) film. However, the film layer 10B is not limited to the above-described types and may be implemented in various ways.

Meanwhile, the adhesive layer 10A may be formed of an adhesive composition, and the adhesive composition may comprise a hydrophilic polymer and an adhesive. That is, in the present invention, the adhesive layer 10A of the adhesive sheet 10 is manufactured using the adhesive composition comprising a hydrophilic polymer.

Therefore, the adhesive layer 10A is hydrophilic, and accordingly, the adhesive sheet 10 to which the microneedles 14 are attached is hydrophilic. When the adhesive sheet 10 or the adhesive layer 10A is hydrophilic, the bonding force between the adhesive sheet 10 and the microneedles 14 may be sufficiently maintained even when the microneedles 14 are formed on the upper surface of the adhesive layer 10A.

According to experiments by the present inventor, it is preferable that the hydrophilic polymer is comprised in the adhesive composition at 5 to 40 wt%. When the hydrophilic polymer is comprised in the adhesive composition at less than 5 wt%, the bonding force between the adhesive layer 10A and the microneedles 14 is weak, making it difficult to form the microneedle patch 2000. In addition, when the hydrophilic polymer is comprised in the adhesive composition at more than 40 wt%, the adhesive force of the adhesive layer 10A becomes very weak, making it difficult to attach the microneedle patch 2000 to the skin of the human body.

For example, the hydrophilic polymer may be composed of one type or a mixture of two or more types selected from CMC (carboxymethyl cellulose), Carbomer, HPMC (hydroxypropyl methyl cellulose), PVP (polyvinyl pyrrolidone), PVA (polyvinyl alcohol), and Guar Gum.

Meanwhile, the aforementioned adhesive may comprise 10 to 50 wt% of synthetic rubber, 30 to 70 wt% of hydrogenated hydrocarbon resin, 5 to 20 wt% of mineral oil, and 0.2 to 3 wt% of an antioxidant.

FIG. 8 corresponds to a photograph of a state in which microneedles are formed on the upper surface of the adhesive sheet 10 comprising a hydrophilic polymer according to the present invention.

Referring to FIG. 8, FIG. 8A corresponds to a case comprising CMC (carboxymethyl cellulose) as the hydrophilic polymer, FIG. 8B corresponds to a case comprising Carbomer as the hydrophilic polymer, FIG. 8C corresponds to a case comprising HPMC (hydroxypropyl methyl cellulose) as the hydrophilic polymer, FIG. 8D corresponds to a case comprising PVP (polyvinyl pyrrolidone) as the hydrophilic polymer, and FIG. 8E corresponds to a case comprising PVA (polyvinyl alcohol) as the hydrophilic polymer.

The present inventor included each of the above-described hydrophilic polymers in the adhesive composition at 5 to 40 wt% and manufactured a microneedle patch by the blowing-stretching method according to FIG. 5.

As shown in FIG. 8, when the microneedle patch 2000 is manufactured using the adhesive sheet 10 according to the present invention, it can be seen that the adhesive sheet 10 and the microneedles 14 are not separated and are normally manufactured.

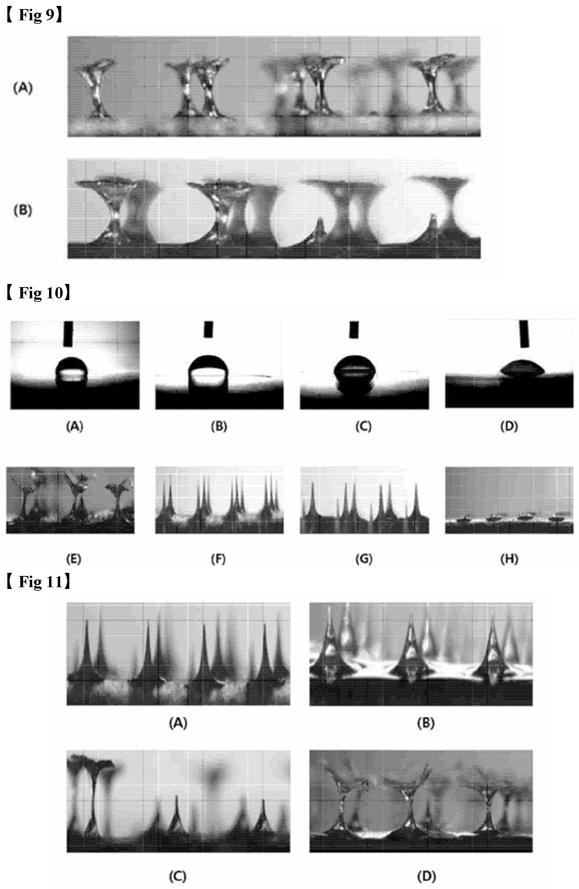

Meanwhile, FIG. 9 corresponds to a photograph of a case in which a microneedle patch is manufactured by the blowing-stretching method according to FIG. 5 when the content of the hydrophilic polymer in the adhesive composition is less than the aforementioned 5 wt%. FIG. 9A is a case in which no hydrophilic polymer is included, and FIG. 9B corresponds to a case in which Guar Gum among the hydrophilic polymers is included at less than 5 wt%.

As shown in FIG. 9, when the hydrophilic polymer included in the adhesive composition is less than the aforementioned 5 wt%, the bonding force between the adhesive layer 10A of the adhesive sheet 10 and the microneedles 14 is weakened. Therefore, when manufacturing the microneedle patch 2000 by the blowing-stretching method according to FIG. 5, it can be seen that the viscous materials 13, 13' (see FIG. 5) are not stretched as described above and are separated from one side of the pair of adhesive sheets 10 and are not formed in the shape of microneedles 14.

Meanwhile, a contact angle of the adhesive sheet 10 according to the present invention may be 70° to 100°. Here, a contact angle may be defined as an angle formed by a liquid-gas interface and a liquid-solid interface when a liquid droplet such as a water droplet exists on a solid.

FIG. 10 is a photograph showing whether the microneedles 14 are formed according to changes in contact angle depending on the content of the hydrophilic polymer in the adhesive sheet 10 according to the present invention and the adhesive sheet according to the prior art when a microneedle patch is manufactured by the blowing-stretching method according to FIG. 5.

FIGS. 10A, 10B, 10C, and 10D show states in which contact angles are measured, and FIGS. 10E, 10F, 10G, and 10H show whether microneedles are formed when a microneedle patch is manufactured by the method according to FIG. 5 according to the corresponding contact angles. For example, FIG. 10E corresponds to FIG. 10A, FIG. 10F corresponds to FIG. 10B, FIG. 10G corresponds to FIG. 10C, and FIG. 10H corresponds to FIG. 10D.

FIG. 10A corresponds to a case in which the content of the hydrophilic polymer is less than 5 wt%, and the measured contact angle corresponds to approximately 100° or more. In this case, as shown in FIG. 10E, it can be seen that a tip portion is not properly formed at the end of the microneedles 14, and the microneedle formation rate corresponds to approximately 20%, which is very low.

In addition, FIG. 10B corresponds to a case in which the content of the hydrophilic polymer is 5 wt% to 20 wt%, and the measured contact angle corresponds to approximately 90° to 100°. In this case, as shown in FIG. 10F, it can be seen that a tip portion is normally formed at the end of the microneedles 14, and the microneedle formation rate corresponds to approximately 100%, indicating that almost all microneedles are normally formed.

Similarly, FIG. 10C corresponds to a case in which the content of the hydrophilic polymer is 20 wt% to 40 wt%, and the measured contact angle corresponds to approximately 70° to 90°. In this case, as shown in FIG. 10G, it can be seen that a tip portion is normally formed at the end of the microneedles 14, and the microneedle formation rate corresponds to approximately 100%, indicating that almost all microneedles are normally formed.

Meanwhile, FIG. 10D corresponds to a case in which the content of the hydrophilic polymer exceeds 40 wt%, and the measured contact angle corresponds to approximately 60° or less. In this case, as shown in FIG. 10H, it can be seen that the microneedles 14 are not properly formed, and the microneedle formation rate corresponds to approximately 40%, which is very low.

Meanwhile, a viscosity of the hydrophilic polymer included in the adhesive composition may be 5 mPa·s to 5,000 mPa·s.

FIG. 11 corresponds to a photograph of the degree of formation of the microneedles 14 depending on the viscosity of the hydrophilic polymer when a microneedle patch is manufactured by the blowing-stretching method according to FIG. 5.

FIG. 11A corresponds to a case in which the viscosity of the hydrophilic polymer is approximately 5 mPa·s to 1,000 mPa·s, and in this case, it can be seen that a tip portion is normally formed at the end of the microneedles 14, and the microneedle formation rate corresponds to approximately 100%, indicating that almost all microneedles are normally formed.

In addition, FIG. 11B corresponds to a case in which the viscosity of the hydrophilic polymer is approximately 3,000 mPa·s to 5,000 mPa·s, and in this case, it can be seen that a tip portion is normally formed at the end of the microneedles 14, and the microneedle formation rate corresponds to approximately 95%, indicating that almost all microneedles are normally formed.

On the other hand, FIGS. 11C and 11D correspond to cases in which the viscosity of the hydrophilic polymer is approximately 10,000 mPa·s to 14,000 mPa·s and approximately 30,000 mPa·s to 45,000 mPa·s, respectively, and in these cases, it can be seen that a tip portion is not normally formed at the end of the microneedles 14, and the microneedle formation rates correspond to approximately 65% and 50%, respectively, indicating that the microneedles are not normally formed.

Although the above description has been made with reference to preferred embodiments of the present invention, those skilled in the art will be able to variously modify and change the present invention within the scope not departing from the spirit and scope of the present invention described in the claims set forth below. Therefore, all modified implementations that basically include the elements of the claims of the present invention should be considered to be included in the technical scope of the present invention.

### [Industrial Applicability]

The present invention relates to a microneedle patch and an adhesive composition, and more particularly, to a microneedle patch and an adhesive composition in which the microneedles may be directly attached to an adhesive sheet without a support film for supporting hydrophilic microneedles when providing the microneedle patch.

## Claims

1. A microneedle patch comprising:
an adhesive sheet; and
hydrophilic microneedles provided on the adhesive sheet,
wherein the microneedles are directly connected to one surface of the adhesive sheet.

2. The microneedle patch according to claim 1, wherein the hydrophilic microneedles are configured as a plurality of microneedles, and the adhesive sheet is exposed between the plurality of microneedles.

3. The microneedle patch according to claim 1 or 2, wherein the adhesive sheet comprises a hydrophilic polymer.

4. The microneedle patch according to claim 3, wherein the adhesive sheet comprises an adhesive layer formed of an adhesive composition comprising the hydrophilic polymer and an adhesive and to which the microneedles are connected, and a film layer provided below the adhesive layer, and the adhesive composition comprises 5 to 40 wt% of the hydrophilic polymer.

5. The microneedle patch according to claim 3, wherein the hydrophilic polymer is composed of one type or a mixture of two or more types selected from CMC (carboxymethyl cellulose), Carbomer, HPMC (hydroxypropyl methyl cellulose), PVP (polyvinyl pyrrolidone), PVA (polyvinyl alcohol), and Guar Gum.

6. The microneedle patch according to claim 4, wherein the adhesive comprises 10 to 50 wt% of synthetic rubber, 30 to 70 wt% of hydrogenated hydrocarbon resin, 5 to 20 wt% of mineral oil, and 0.2 to 3 wt% of an antioxidant.

7. The microneedle patch according to claim 3, wherein a contact angle of the adhesive sheet is 70° to 100°.

8. The microneedle patch according to claim 3, wherein a viscosity of the hydrophilic polymer is 5 mPa·s to 5,000 mPa·s.

9. An adhesive composition for forming an adhesive layer of an adhesive sheet included in a microneedle patch, wherein the composition comprises a hydrophilic polymer.

10. The adhesive composition according to claim 9, wherein the hydrophilic polymer is composed of one type or a mixture of two or more types selected from CMC (carboxymethyl cellulose), Carbomer, HPMC (hydroxypropyl methyl cellulose), PVP (polyvinyl pyrrolidone), PVA (polyvinyl alcohol), and Guar Gum.

11. The adhesive composition according to claim 9, wherein the hydrophilic polymer is comprised in the composition at 5 to 40 wt%.

12. The adhesive composition according to claim 9, wherein the composition comprises an adhesive and the hydrophilic polymer, and the adhesive comprises 10 to 50 wt% of synthetic rubber, 30 to 70 wt% of hydrogenated hydrocarbon resin, 5 to 20 wt% of mineral oil, and 0.2 to 3 wt% of an antioxidant.

13. The adhesive composition according to claim 9, wherein a viscosity of the hydrophilic polymer is 5 mPa·s to 5,000 mPa·s.
